(19) Europäisches Patentamt
European Patent Office
Office européen des brevets

(11) Veröffentlichungsnummer: **0 291 862**
**A1**

(12) # EUROPÄISCHE PATENTANMELDUNG

(21) Anmeldenummer: **88107665.7**

(51) Int. Cl.⁴: **C07D 495/04 , A61K 31/55**

(22) Anmeldetag: **13.05.88**

Die Anmeldung wird, wie ursprünglich eingereicht, unvollständig veröffentlicht (Art. 93 (2) EPÜ). Die Stelle der Patentansprüche, die offensichtlich eine Auslassung enthält, ist als Lücke an der entsprechenden Stelle ersichtlich (Patentansprüche für Spanien sind nicht eingereicht worden).

(30) Priorität: **21.05.87 DE 3717069**

(43) Veröffentlichungstag der Anmeldung:
**23.11.88 Patentblatt 88/47**

(84) Benannte Vertragsstaaten:
**AT BE CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Steiner, Gerd, Dr.**
**Oberer Waldweg 1**
**D-6719 Kirchheim(DE)**
Erfinder: **Teschendorf, Hans-Juergen, Dr.**
**Georg-Nuss-Strasse 5**
**D-6724 Dudenhofen(DE)**
Erfinder: **Unger, Liliane, Dr.**
**Waltraudenstrasse 14**
**D-6700 Ludwigshafen(DE)**
Erfinder: **Binder, Rudolf, Dr.**
**Ostpreussenstrasse 1**
**D-6520 Worms(DE)**

(54) **5-substituierte 10-Cyanmethylenthieno [3,4-b]-benzo-azepine.**

(57) Es werden Verbindungen der Formel

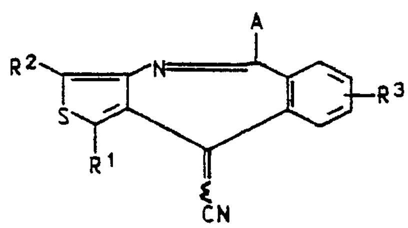

I,

worin R¹, R², R³ und A die in der Beschreibung angegebene Bedeutung besitzen, sowie deren Verwendung bei der Bekämpfung von Krankheiten beschrieben.

EP 0 291 862 A1

## 5-substituierte 10-Cyanmethylen-thieno[3,4-b]-benzo-azepine

Die Erfindung betrifft in 5-Stellung substituierte 10-Cyanmethylenthieno[3,4-b]-benzo-azepine, Verfahren zu ihrer Herstellung und ihre Anwendung als Arzneimittel, die als Sedativa, Hypnotika, Tranquilizer, Muskelrelaxantien, Neuroleptika oder Antiparkinsonmittel verwendet werden können.

Es ist bekannt, daß tricyclische Ringsysteme mit einer Dibenzo-Struktur zu einem zentralen heterocyclischen 7-Ring, der gegebenenfalls einen basischen Seitenrest, wie beispielsweise einen N-Methylpiperazinrest aufweist, neuroleptische Wirkungen aufweisen können. Solche Tricyclen sind beispielsweise N-Methylpiperazin-Derivate von Dibenzo[b,e,][1,4]diazepinen (Clozapine), Dibenzo[b,f][1,4]-thiazepinen (Clotiapine), Dibenzo-[b,f][1,4]-oxazepinen (Loxapine) oder Morphantridinen (Perlapine), wie beispielsweise aus der Zusammenfassung von J. Schmutz in der Arzneimittelforschung 25, 712-720 (1975) hervorgeht.

In der DE-OS 29 18 778, der DE-OS 30 37 971 und der DE-OS 35 24 744 werden 6-substituierte 11-Alkylen-morphantridine, 5-substituierte-9Cyanmethylen-dithieno-[3,4-b:4′.3′-e]azepine und 4-substituierte 10-Cyanmethylen-thieno[4,3-e]benzo-azepine mit wertvollen pharmakologischen Eigenschaften beschrieben.

Es wurde nun gefunden, daß 5-substituierte 10-Cyanmethylenthieno[3,4-b]-benzo-azepine der Formel I

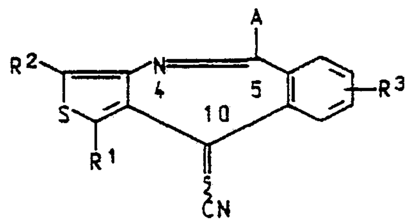

I,

in der

$R^1$, $R^2$, $R^3$ Wasserstoff- oder Halogenatome oder Alkylreste mit 1 bis 3 C-Atomen bedeuten,

A für einen Aminorest $-NR^4R^5$ steht, in dem $R^4$ und $R^5$ zusammen mit dem sie verbindenden Stickstoffatom einen 5- bis 7-gliedrigen gesättigten Ring bedeuten, der gegebenenfalls als weiteres Heteroatom ein Stickstoff- oder Sauerstoffatom enthält, wobei ein zusätzlich vorhandenes Stickstoffatom gegebenenfalls durch einen Alkylrest mit 1 bis 3 C-Atomen, einen Hydroxyalkylrest mit 2 bis 3 C-Atomen, einen Alkoxyalkylrest mit 2 bis 3 C-Atomen im Alkyl- oder Alkoxyrest, einen Cycloalkyl- oder Cycloalkylmethylrest mit 3 bis 7 C-Atomen im Cycloalkylring, einen Alkinylrest mit 2 bis 5 C-Atomen und gegebenenfalls zusätzlich durch Sauerstoff in Form eines N-Oxids substituiert ist, oder A einen Aminorest $-NHR^6$, in dem $R^6$ einen Aminoalkylrest mit 2 bis 7 C-Atomen bedeutet, wobei das Aminstickstoffatom gegebenenfalls durch einen niederen Alkylrest mit 1 bis 5 C-Atomen substituiert oder Bestandteil eines 5- bis 7-gliedrigen gesättigten Ringes ist, der gegebenenfalls ein Stickstoff- oder Sauerstoffatom als weiteres Heteroatom enthält, wobei ein vorhandenes Stickstoffatom durch einen niederen Alkylrest mit 1 bis 3 C-Atomen oder einen Hydroxyalkylrest mit 2 bis 3 C-Atomen substituiert sein kann, bedeutet, und ihre physiologisch verträglichen Säureadditionssalze wertvolle pharmakologische Eigenschaften aufweisen.

Für die Reste $R^1$, $R^2$, $R^3$ kommen insbesondere folgende Bedeutungen in Betracht: Wasserstoff, Fluor, Chlor und Methyl.

Als Aminreste $-NR^4R^5$ für A sind vorzugsweise Piperazinyl-, Homopiperazinyl-, Piperidinyl- und Morpholinylreste zu nennen.

Besonders bevorzugte Reste $-NR^4R^5$ sind der 4-Methyl-piperazinyl-, 4-Methyl-4-oxy-piperazinyl-, 4-Ethyl-piperazinyl- und der N-Methyl-homopiperazinyl-Rest.

Im Aminrest $-NHR^6$ sind für $R^6$ der 2-Dimethylamino-ethyl- und der 2-Piperidin-1-yl-ethyl-Rest hervorzuheben.

Es sei darauf hingewiesen, daß die erfindungsgemäßen Verbindungen der Formel I als (E)- bzw. (Z)-Isomere Ia und b vorliegen.

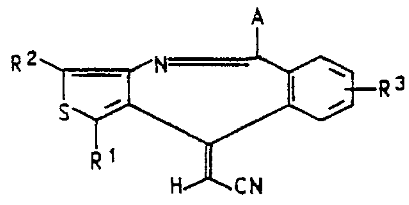

Ia

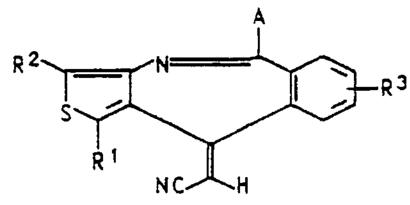

Ib

Die (E), (Z)-Isomeren können beispielsweise durch fraktionierte Kristallisation oder durch Säulenchromatograhie getrennt werden.

Die folgenden Verbindungen sind als besonders bevorzugt zu nennen:
(E),(Z)-10-Cyanmethylen-5-(4-methyl-piperazin-1-yl)-thieno[3,4-b]benzoazepin
(E)-10-Cyanmethylen-5-(4-methyl-piperazin-1-yl)-thieno[3,4-b]benzo-azepin
(Z)-10-Cyanmethylen-5-(4-methyl-piperazin-1-yl)-thieno[3,4-b]benzo-azepin

Wie die Ausführungsbeispiele zeigen, kann im Einzelfall die Trennung in das (E)- und (Z)-Isomere ohne allzu großen Aufwand vorgenommen werden.

Die erfindungsgemäßen Verbindungen der Formel I werden hergestellt, indem man eine Verbindung der Formel II

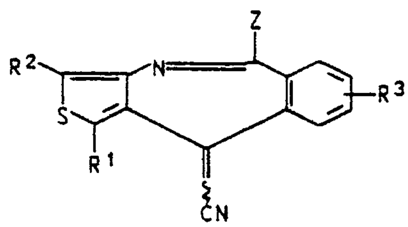

II,

in der $R^1$, $R^2$, $R^3$ die angegebenen Bedeutungen haben und Z eine nukleofuge Abgangsgruppe darstellt, mit einem Nukleophil AH, in dem A die für Formel I angegebenen Bedeutungen hat, umsetzt, gegebenenfalls in das reine cis- und trans-Isomere trennt und/oder gegebenenfalls die erhaltene Verbindung in das N-Oxid und/oder in das Säureadditionssalz einer physiologisch verträglichen Säure überführt.

Als nukleofuge Abgangsgruppe für Z kommen Halogenatome, insbesondere Brom oder Chlor, in Betracht. Die Umsetzung erfolgt zweckmäßig in Gegenwart einer überschüssigen Menge des verwendeten Amins AH, das gleichzeitig als Lösungsmittel und gegebenenfalls als säurebindendes Mittel dient. Gegebenenfalls kann in Gegenwart eines inerten Lösungsmittels, wie einem cyclischen gesättigten Ether, insbesondere Tetrahydrofuran oder Dioxan, von Benzol oder eines Benzolkohlenwasserstoffes, wie Toluol, Xylol, Mesitylen oder Decahydronaphthalin oder eines aprotischen polaren Lösungsmittels, wie Dimethylformamid, gearbeitet werden. Falls nur mit einem Äquivalent des Amins AH gearbeitet wird, muß noch 1 Äquivalent einer inerten Base, wie z.B. Triethylamin, zugesetzt werden.

Die Umsetzung erfolgt in der Regel bei Temperaturen von 80 bis 150°C, und ist im allgemeinen innerhalb von 1 bis 10 Stunden beendet. Gegebenfalls ist der Ausschuß des Sauerstoffs der Luft und das Arbeiten unter Inertgas, beispielsweise unter Stickstoff, von Vorteil.

Bei den Umsetzungen wird das Nukleophil AH vorteilhafterweise in mindestens 2- bis 20-fachem molarem Überschuß verwendet.

Die Überführung einer Verbindung der Formel I in das N-Oxid erfolgt in üblicher Weise, zweckmäßig mit wäßrigem Wasserstoffperoxid (30 Gew.%) in ethanolischer Lösung. Die Überführung in das Säureadditionssalz einer physiologisch verträglichen Säure erfolgt ebenso in üblicher Weise.

Die Ausgangsverbindungen der Formel II werden erhalten, indem man ein 10-Cyanmethylen-4,5-dihydro-thieno[3,4-b]benzo-azepin-5-on der Formel III

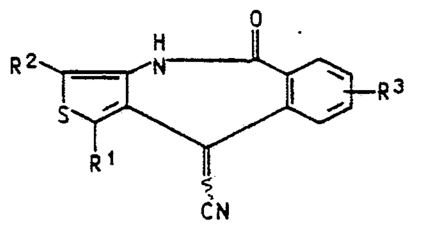

III,

in der $R^1$, $R^2$, $R^3$ die für Formel II angegebenen Bedeutungen hat, mit einem Überschuß an Phosphoroxychlorid, in Gegenwart eines Lösungsmittels, vorzugsweise einem Halogenkohlenwasserstoff, und in Gegenwart einer katalytischen Menge N,N-Dimethylanilin 1 bis 5 Stunden auf Rückflußtemperatur erhitzt und das erhaltene Iminochlorid nach dem Abdestillieren des überschüssigen Phosphoroxychlorids und Aufarbeitung in einem wäßrigen zweiphasigen System durch Extraktion mit Methylenchlorid isoliert.

Das neue 10-Cyanmethylen-4,5-dihydro-thieno[3,4-b]benzo-azepin-5-on der Formel III, in der $R^1$, $R^2$, $R^3$ die für Formel I angegebenen Bedeutungen hat, wird durch Carbonyl-Olefinierung hergestellt, indem man ein 4,5-Dihydro-thieno[3,4-b]benzo-azepin-5,10-dion der Formel IV

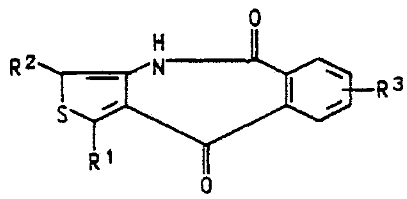

IV,

mit einem Phosphonat der Formel Va

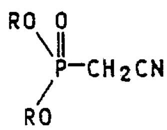

Va,

in der R einen Alkylrest mit 1 bis 3 C-Atomen bedeutet, unter den Bedingungen der Witting-Horner-Reaktion in Dimethylformamid in Gegenwart von einem Moläquivalent einer Base, wie z.B. Natriumalkoholat, bei Temperaturen von 20 bis 80°C
oder mit einem Phosphoniumsalz der Formel Vb

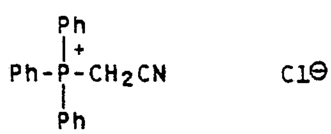

Vb,

in der Ph für einen Phenylrest steht, unter den Bedingungen der klassischen Wittig-Reaktion in Dimethylformamid in Gegenwart von einem Moläquivalent einer starken Base bei Temperaturen von 20 bis 100°C umsetzt.

Das neue 4,5-Dihydro-thieno[3,4-b]benzo-azepin-5,10-dion der Formel IV, in der $R^1$, $R^2$ $R^3$ die für die Formel I angegebenen Bedeutungen hat, wird durch Friedel-Crafts-Cyclisierung hergestellt, indem man ein 2′5′-disubstituiertes Monophthalsäure-thiophen-3-amid der Formel VI

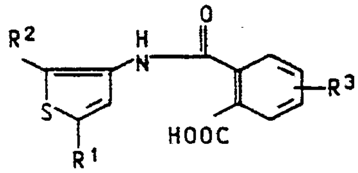

VI

in der $R^1$ und $R^2$ die für die Formel I angegebenen Bedeutungen, ausgenommen Wasserstoff, haben, zunächst mit Thionylchlorid in einem Chlorkohlenwasserstoff oder in überschüssigem Thionylchlorid ohne Verwendung eines Lösungsmittels bei Temperaturen von 20 bis 80 °C in das entsprechende Säurechlorid überführt und dieses anschließend in Gegenwart von 1 bis 1,5 Moläquivalent Aluminiumchlorid in einem inerten organischen Lösungsmittel, wie z.B. einem Chlorkohlenwasserstoff, oder einem dipolaren aprotischen Lösungsmittel, wie z.B. Dimethylformamid, bei Temperaturen von 20 bis 100 °C cyclisiert.

Zur Überführung in das 4,5-Dihydro-thieno[3,4-b]-benzo-azepin-5,10-dion der Formel IV mit $R^1$ und $R^2$ = H entfernt man im Fall $R^1$ und $R^2$ = Chlor die Halogensubstituenten durch eine katalytische Hydrierung in Gegenwart eines Edelmetallkatalysators, beispielsweise Palladium auf Kohlenstoff, in einem inerten organischen Lösungsmittel, vorzugsweise N-Methylpyrrolidon oder Dimethylformamid, unter Zusatz eines Säurefängers wie Natriumacetat oder Natriumhydroxyd, bei Drücken zwischen Normaldruck und 100 bar und Temperaturen zwischen 20 und 100 °C.

Das 2′,5′-disubstituierte Monophthalsäure-thiophen-3-amid der Formel VI erhält man in einfacher Weise durch Umsetzung eines 2,5-disubstituierten3-Aminothiophens (GB-PS 13 34 015) mit einem Phthalsäureanhydrid in einem inerten organischen Lösungsmittel wie Toluol oder Tetrahydrofuran bei Raumtemperatur während 1 bis 5 Stunden.

Die erfindungsgemäßen Verbindungen der Formel I werden in der Regel in Form gelblicher bis gelber Kristalle erhalten und können durch Umkristallisation aus den üblichen organischen Lösungsmitteln, bevorzugt aus einem niederen Alkohol, wie Ethanol, umkristallisiert oder durch Säulenchromatographie gereinigt werden.

Falls erforderlich erfolgt eine Trennung in die einzelnen cis- und trans-Isomeren durch fraktionierte Kristallisation in einem chlorierten Kohlenwasserstoff, bevorzugt Methylenchlorid, einem niederen einwertigen Alkohol, bevorzugt Methanol oder Ethanol, oder einem gesättigten cycloaliphatischen Kohlenwasserstoff, bevorzugt Cyclohexan, oder durch Säulenchromatographie, insbesondere in Methylenchlorid und Methanol im Verhältnis 99:1 bis 85:15 Volumenteilen.

Die freien substituierten 10-Cyanmethylen-thieno[3,4-b]benzo-azepine der Formel I können in üblicher Weise in das Säureadditionssalz einer pharmakologisch verträglichen Säure überführt werden, vorzugsweise durch Versetzen einer Lösung mit einem Äquivalent der entsprechenden Säure. Pharmazeutisch verträgliche Säuren sind beispielsweise Salzsäure, Phosphorsäure, Schwefelsäure, Methansulfonsäure, Amidosufonsäure, Maleinsäure, Fumarsäure, Oxalsäure, Weinsäure und Zitronensäure.

Die erfindungsgemäßen Verbindungen weisen wertvolle pharmakologische Eigenschaften auf. Sie können als Sedativa, Hypnotika, Tranquilizer, Muskelrelaxantien, Neuroleptika oder Antiparkinsonmittel Vewendung finden. Mehrere der genannten Wirkungsqualitäten können kombiniert bei einer erfindungsgemäßen Verbindung auftreten. In manchen Fällen kann das einzelne reine Isomere nach erfolgter Isomerentrennung eine Wirkung bevorzugt aufweisen. Die neuen Verbindungen eignen sich daher zur Behandlung psychischer Störungen, insbesondere Schizophrenie, Angst, Unruhezustände sowie Störungen des extrapyramidalmotorischen Systems, z.B. Morbus Parkinson.

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln, sowie die Verwendung der neuen Verbindungen bei der Bekämpfung von Krankheiten.

Die erfindungsgemäßen Verbindungen können in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 20 mg/kg Körpergewicht bei oraler Gabe und zwischen 0,1 und 2 mg/kg Körpergewicht bei parenteraler Gabe.

Die neuen Verbindungen können in gebräuchlichen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 0,1 bis 99 Gew.%.

Die folgenden Beispiele dienen zur Erläuterung der Erfindung:

Beispiel 1

A. Herstellung der Ausgangsstoffe

a) Monophthalsäure-2,5-dichlor-thiophen-3-amid

40,0 g (196 mM) 2,5-Dichlor-3-amino-thiophen-Hydrochlorid wurden in 300 ml Toluol und 500 ml Wasser unter intensivem Rühren mit 2,5 N Natronlauge auf pH = 10 gestellt. Nach der Phasentrennung saugte man die unlöslichen Anteile ab und ließ nach dem Trocknen die Toluollösung des freien Amins zu 29,0 g (196 mM) Phthalsäureanhydrid in 100 ml Toluol bei Raumtemperatur unter gutem Rühren zutropfen. Man ließ anschließend 2 bis 3 h nachrühren, saugte nach dem Kühlen mit Eis die dick ausgefallenen Festkörper ab, wusch gut mit Toluol nach und trocknete die Festkörper zuerst an der Luft und später im Vakuumtrockenschrank. Man erhielt 57,6 g (93 %), Schmp. 169-171 °C.

b) 1,3-Dichlor-4,5-dihydro-thieno[3,4]benzo-azepin-5,10-dion

Zu 30,0 g (95 mM) Mopnophthalsäure-2,5-dichlor-thiophen-3-amid in 450 ml Chloroform wurden unter Rückflußkochen 20,0 ml (260 mM) Thionylchlorid unter Rühren innerhalb 0,5 h getropft. Man ließ den Ansatz eine weitere 0,5 h am Rückfluß kochen und engte dann den Ansatz im Vakuum zur Trockene ein. Das zurückbleibende Säurechlorid (29,0 g) wurde vorsichtig in eine Suspension von 120 g Aluminiumchlorid in 18 ml Dimethylformamid bei 90 °C unter intensivem Rühren portionsweise während 10 bis 15 min zugegeben. Anschließend ließ man den Ansatz noch 30 min bei 90 °C nachrühren und goß vorsichtig auf 2 l Eis/Wasser. Man versetzte mit 20 ml konzentrierter Salzsäure, rührte die wäßrige Suspension noch 1 h nach und saugte die hellbraunen Festkörper ab. Nach dem Nachwaschen mit $H_2O$ wurde das Produkt im Vakuumtrockenschrank bei 60 °C getrocknet. Man erhielt 27,8 g (98 %) Produkt mit genügender Reinheit für die weitere Umsetzung, Schmp. > 250 °C..

c1) 4,5-Dihydro-thieno[3,4-b]benzo-azepin-5,10-dion

10,0 g (34 mM) 1,3-Dichlor-4,5-dihydro-thieno[3,4-b]benzo-azepin5,10-dion in 450 ml 1-Methyl-pyrrolidon-2 wurden mit 2,1 g Pd/C (10 %) sowie mit 7,4 g (90 mM) fein pulverisiertem Natriumacetat versetzt und 10 h unter Normaldruck bei 50-60 °C hydriert. Anschließend saugte man ab und wusch mit Pyrrolidon, dann mit $H_2O$ nach. Dann goß man das Filtrat auf 2 l Eiswasser, säuerte mit konzentrierter Salzsäure an, saugte nach 1 h Nachrühren die Festkörper unter gutem Nachwaschen mit $H_2O$ ab und trocknete das Rohprodukt (3,7 g) im Vakuum.

c2) Variante für die halogensubstituierten Derivate

7(8)-Chlor-4,5-dihydro-thieno[3,4-b]benzo-azepin-5,10-dion

6,0 g (18mM) 1,3,7(8)-Trichlor-4,5-dihydro-thieno[3,4-b]benzo-azepin-5,10-dion in 200 ml n-Butanol wurden mit 10,0 g (158 mM) Ammoniumformiat und 2,5 g Palladium auf Kohle (10 %) versetzt und 4 h unter Rückfluß gekocht. Nach Einengen zur Trockene nahm man den Rückstand in 150 ml $H_2O$ auf, säuerte mit konzentrierter Salzsäure an, saugte den Niederschlag nach einstündigem intensiven Rühren ab, wusch mehrmals mit $H_2O$ nach und trocknete im Vakuumtrockenschrank. Das Rohprodukt wurde in 150 ml DMF aufgenommen und bei 100 °C dispergiert. Die Festkörper saugte man in der Hitze ab und wusch mit heißem DMF nach. Man isolierte 4,1 g (72 %) Produkt, das aus Eisessig umkristallisiert wurde. Schmp.: 298-302 °C

d) (E),(Z)-10-Cyanmethylen-4,5-dihydro-thieno[3,4-b]benzo-azepin-5-on (E),(Z)-Isomerengemisch

Zur Herstellung dieses Produktes führte man eine Carbonyl-Olefinierung des 4,5-Dihydro-thieno[3,4-b]-benzo-azepin-5,10-dions mit Hilfe der Wittig-Horner-Reaktion (α) oder über die klassische Wittig-Synthese (β) durch:

α) 10,0 g (43,7 mM) 4,5-Dihydro-thieno[3,4-b]benzo-azepin-5-10-dion wurden in 130 ml Dimethylformamid unter Erwärmen gelöst und unter Stickstoff gerührt. Man ließ dann gleichzeitig 9,5 g (53 mM) Diethyl-cyanmethyl-phosphonat und 9,5 g (55 mM) Natriummethylat (30 %) gelöst in 10 ml Dimethylformamid langsam zutropfen (Farbzunahme und Temperaturerhöhung zeigen den Beginn der Wittig-Reaktion an). Nach 12 h Nachrühren bei Raumtemperatur goß man das Reaktionsprodukt auf Eiswasser, säuerte mit konzentrierter Salzsäure an und saugte die ausfallenden Festkörper ab. Nach gutem Nachwaschen mit Wasser wurde das Produkt im Vakuum getrocknet. Ausbeute: 9,7 g (88%) 10-Cyanmethylen-4.5-dihydro-thieno[4,3-e]benzo-azepin-4-on.

β) Man legte Triphenyl-cyanomethyl-phosphonium-chlorid in Dimethylformamid vor, ließ anschließend 1 Moläquivalent einer 30-%igen Natriummethylat-Lösung zutropfen oder versetzte mit 1 Moläquivalent Natriumhydrid und fügte zuletzt 1 Moläquivalent einer Lösung von 4,5-Dihydro-thieno[4,3-e]benzo-azepin-4,10-dion in Dimethylformamid hinzu. Man ließ das Reaktionsgemisch 5 bis 8 h bei 50 bis 80 °C nachrühren, goß anschließend auf Eiswasser und extrahierte mehrmals mit Methylenchlorid. Nach dem Trocknen und Einengen der organischen Phase kristallisierte man das Rohprodukt aus Ethanol um. Ausbeute: 71 % farblose Kristalle.

In analoger Weise erhielt man (E), (Z)-1,3-Dichlor-10-cyanmethylen4,5-dihydro-thieno[3,4-b]benzo-azepin-5-on durch Einsatz von 1,3-Dichlor-4,5-dihydro-thieno[3,4-b]benzo-azepin-5,10-dion in die Wittig-Horner-Reaktion und Erhöhung der Reaktionstemperatur auf 50 bis 70 °C , Schmp. > 250 °C.

B. Herstellung der Endprodukte

(E)- und (Z)-10-Cyanmethylen-5-(4-methyl-piperazin-1-yl)thieno[3,4-b]-benzo-azepin

a) 9,6 g (38 mM) 10-Cyanmethylen-4,5-dihydro-thieno[3,4-b]benzoazepin-5-on ((E),(Z)-Isomerengemisch) in 60 ml 1,1,2-Trichlorethan wurden mit 30 ml Phosphoroxychlorid und 0,3 ml N,N-Dimethylanilin versetzt und 1,5 h unter Stickstoff unter Rückfluß gekocht. Nach vollständigem Abdestillieren des überschüssigen Phosphoroxychlorids und Dimethylanilins im Ölpumpenvakuum verteilte man den Rückstand zwischen Methylenchlorid und Wasser, extrahierte die wäßrige Phase noch zweimal mit Methylenchlorid und wusch die vereinigten organischen Phasen mit verdünnter HCl und Wasser gründlich nach. Trocknen und Einengen der organischen Phase lieferte 10,2 g (99 %) 5-Chlor-10cyanmethylen-thieno[3,4-b]benzo-azepin, das für die weitere Umsetzung genügend rein ist.

10,2 g (37 mM) 5-Chlor-10-cyanmethylen-thieno[3,4-b]benzo-azepin wurden in 120 ml Dimethylformamid gelöst, mit 10 ml (90 mM) N-Methylpiperazin (stark exotherme Reaktion) versetzt und 2 bis 3 h bei 100 °C unter Stickstoff gerührt. Nach Entfernen des Lösungsmittels im Vakuum nahm man den Rückstand in 300 ml Eis/Wasser auf, stellte mit wenig 2,5 N Natronlauge alkalisch, ließ 1 h unter Kühlung nachrühren und saugte das hellbraune Rohprodukt unter reichlichem Nachwaschen mit Wasser ab. Anschließend verteilte man das Rohprodukt zwischen Methylenchlorid und Wasser und arbeitete die organische Phase wie üblich durch Trocknen und Einengen auf. Das Rohprodukt reinigte man durch Säulenchromatographie (Kieselgel, Laufmittel Methylenchlorid/Methanol 95/5). Man erhielt 8,0 g (66 %) gelbliches 10-Cyanmethylen-4-(4-methyl-piperazin-1-yl)-thieno[4,3-e]benzo-azepin in Form eines (E),(Z)-Isomerengemisches, Schmp. 146 bis 150 °C.

b) Zur Trennung der (E),(Z)-Isomeren digerierte man 4,0 g Isomerengemisch in 40 ml Toluol/Ethanol 3/1 und saugte nach 1 h die nicht gelösten Kristalle ab.

Man isolierte als erste Fraktion 1,2 g gelbe Kristalle, die nach dem Dünnschichtchromatogramm (Kieselgel, Laufmittel Toluol/Methanol 85/15) vornehmen aus dem polaren (Z)-Isomeren b bestehen.

Aus dem Filtrat kristallisierten, nachdem man die Lösung durch Einengen etwas konzentriert hatte, langsam 1,3 g gelbe Kristalle, die nach dem Dünnschichtchromatogramm (Kieselgel, Laufmittel Toluol/Methanol 85/15) vornehmlich aus dem unpolaren (E)-Isomeren a bestehen.

Durch Einengen der Mutterlauge und Umkristallisieren des Rückstandes aus Ethanol erhielt man noch zusätzlich 0,5 g des angereicherten polaren (Z)-Isomeren b.

Durch darauffolgende ein- bis zweimalige Kristallisation der angereicherten Produkte a und b aus Ethanol fielen die (E),(Z)-Isomeren rein an.

Schmp.: (E)-Isomeres a 154-156°C. (Z)-Isomeres b 172 - 173°C .

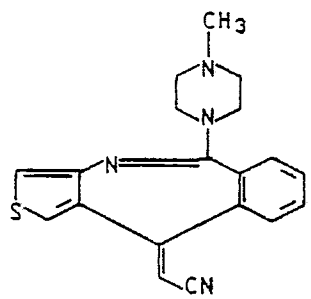

a

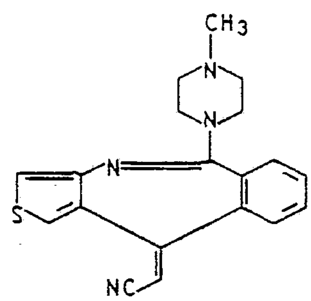

b

Beispiel 2

(E),(Z)-10-Cyanmethylen-5-(4-methyl-4-oxy-piperazin-1-yl)-thieno-[3,4-b]benzo-azepin • $2H_2O$.

3,3 g (10 mM) cis, trans-10-Cyanmethylen-2-methyl-4-(4-methylpiperazin-1-yl)-pyrrolo-[4,3-e]benzo-azepin (vgl. Beispiel 1) wurden in 100 ml Methylenchlorid gelöst und mit 2,2 g (10 mM) 3-Chlorperoxybenzoesäure versetzt. Nach 1 h Nachrühren bei Raumtemperatur engte man den Ansatz ein und reinigte das erhaltene N-Oxid durch Säulenchromatographie (Kieselgel, Laufmittel Methylenchlorid/Methanol 1:1). Man isolierte 2,8 g (80 %) gelbe Kristalle, Schmp. 105-108 °C (Zersetzung).

Die folgenden Substanzen erhielt man analog Beispiel 1 und 2 unter Einsatz der entsprechenden substituierten Ausgangsverbindungen:

3. (E),(Z)-1,3-Dichlor-10-cyanmethylen-5-(4-methyl-piperazin-1-yl)thieno[3,4-b]benzo-azepin.

4. (E),(Z)-7,8-Dichlor-10-cyanmethylen-5-(4-methyl-piperazin-1-yl)-thieno[3,4-b]benzo-azepin.

5a. (E),(Z)-7-Chlor-10-cyanmethylen-5-(4-methyl-piperazin-1-yl)-thieno[3,4-b]benzo-azepin.

Da die Herstellung von 5a analog Beispiel 1Aa bis 1 B ausgehend von m-Chlorphthalsäureanhydrid erfolgte, entstand neben dem 7-Chlor-Derivat auch das 8-Chlor-Derivat. Die Trennung durch Säulenchromatographie (Kieselgel, Laufmittel Toluol/Ethanol = 85/15) lieferte das E-Isomere von 5a, Schmp. 157-159 °C (Ethanol) (unpolare Komponente) [$^{13}C$ NMR, 90 MHz, (DMSO-$D_6$) $\delta$ 45,73 ($N-CH_3$); 47,22 und 54,19 (Piperazin $CH_2$); 98,06 (=$\underline{C}$H-CN); 115,36 (3-C); 116,99 (Nitril); 122,33 (6-C); 129,50 (9-C); 129,85 (1-C); 131,34 (8-C); 134,72 (10a-$\underline{C}$); 135,83 (7-C); 136,56 (9a-C); 143,73 (10-C); 154,38 (3a-C); 156,89 (5-C)] und das Z-Isomere, Schmp. 234-236 °C (Ethanol) (polare Komponente) sowie

5b. (E),(Z)-8-Chlor-10-cyanmethylen-5-(4-methyl-piperazin-1-yl)thieno[3,4-b]benzo-azepin.

6. (E),(Z)-10-Cyanmethylen-5-(4-ethyl-piperazin-1-yl)-thieno[3,4-b]benzo-azepin.

7. (E),(Z)-10-Cyanmethylen-5-(2-piperidin-1-yl)-ethyl-amino)-thieno[3,4-b]benzo-azepin.

8. (E),(Z)-10-Cyanmethylen-5-(2-dimethylamino-ethylamino)-thieno[3,4-b]-benzo-azepin.

Beispiel 9

Auf einer Tablettenpresse werden in üblicher Weise Tabletten folgender Zusammensetzung gepreßt:

40 mg Substanz des Beispiels 1 (E)

120 mg Maisstärke

13,5 mg Gelatine
45 mg Milchzucker
2,25 mg Aerosil® (chemisch reine Kieselsäure in submikroskopisch feiner Verteilung)
6,75 mg Kartoffelstärke (als 6 %iger Kleister)

Beispiel 10

In üblicher Weise werden Dragees folgender Zusammensetzung hergestellt:
20 mg Substanz des Beispiels 1 (E)
60 mg Kernmasse
60 mg Verzuckerungsmasse

Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

Beispiel 11

10 g Substanz des Beispiels 1 (E) werden als Hydrochlorid in 5000 ml Wasser unter Zusatz von NaCl gelöst und auf pH 6,0 eingestellt, so daß eine blutisotonische Lösung entsteht. Jeweils 5 ml dieser Lösung werden in Ampullen gefüllt und sterilisiert.

**Ansprüche**

1. 5-substituierte 10-Cyanmethylen-thieno[3,4-b]benzo-azepine der Formel I

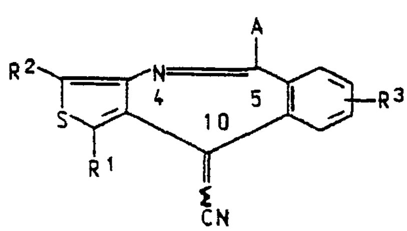

I,

in der
$R^1$, $R^2$, $R^3$ Wasserstoff- oder Halogenatome oder Alkylreste mit 1 bis 3 C-Atomen bedeuten, und
A für einen Aminorest $-NR^4R^5$ steht, in dem $R^4$ und $R^5$ zusammen mit dem sie verbindenen Stickstoffatom einen 5- bis 7-gliedrigen gesättigten Ring bedeuten, der gegebenenfalls als weiteres Heteratom ein Stickstoff- oder Sauerstoffatom enthält, wobei ein zusätzlich vorhandenes Stickstoffatom gegebenenfalls durch einen Alkylrest mit 1 bis 3 C-atomen, einen Hydroxyalkylrest mit 2 bis 3 C-Atomen, einen Alkoxyalkylrest mit 1 bis 3 C-Atomen im Alkyl- und Alkoxyrest, einen Cycloalkyl- oder Cycloalkylmethylrest mit 3 bis 7 C-Atomen im Cycloalkylring, einen Alkinylrest mit 2 bis 5 C-Atomen und gegebenenfalls zusätzlich durch Sauerstoff in Form eines N-Oxids substituiert ist, oder A einen Aminorest $-NHR^6$, in dem $R^6$ einen Aminoalkylrest mit 2 bis 7 C-Atomen bedeutet, wobei das Aminstickstoffatom gegebenenfalls durch einen niederen Alkylrest mit 1 bis 5 C-Atomen substituiert oder Bestandteil eines 5- bis 7-gliedrigen gesättigten Ringes ist, der gegebenenfalls ein Stickstoff- oder Sauerstoffatom als weiteres Heteroatom enthält, wobei ein vorhandenes Stickstoffatom durch einen niederen Alkylrest mit 1 bis 3 C-Atomen oder einen Hydroxyalkylrest mit 2 bis 3 C-Atomen substituiert sein kann, bedeutet, und ihre physiologisch verträglichen Säureadditionssalze.

2. Verbindungen der Formel I nach Anspruch 1, in denen R Wasserstoff, Chlor oder Fluor bedeutet und A für Piperidin, Piperazin oder Homopiperazin steht, die am gegebenenfalls vorhandenen Ringstickstoffatom durch Wasserstoff, Methyl, Ethyl, ß-Hydroxyethyl, Cyclopropyl oder Propinyl substituiert sind und/oder gegebenenfalls in Form des N-Oxids vorliegen können.

3. (E),(Z)-10-Cyanmethylen-5-(4-methyl-piperazin-1-yl)thieno[3,4-b]benzo-azepin.

4. (E)-10-Cyanmethylen-5-(4-methyl-piperazin-1-yl)-thieno[3,4-b]benzoazepin.

5. (Z)-10-Cyanmethylen-5-(4-methyl-piperazin-1-yl)-thieno[3,4-b]benzoazepin.

6. (E),(Z)-7-Chlor-10-Cyanmethylen-5-(4-methyl-piperazin-1-yl)thieno[3,4-b]benzo-azepin.

7. Verfahren zur Herstellung von Verbindungen der Formel I, dadurch gekennzeichnet, daß man eine Verbindung der Formel II

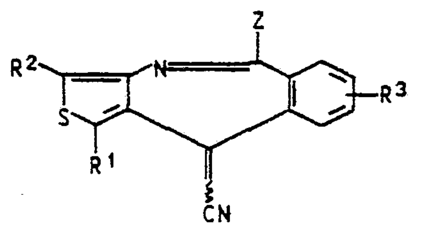 II.

in der $R^1$, $R^2$, $R^3$ die für Formel I angegebenen Bedeutungen hat und Z eine nukleofuge Abgangsgruppe darstellt, mit einem Amin AH, in dem A die für Formel I angegebenen Bedeutungen hat, umsetzt und gegebenenfalls die erhaltene Verbindung in das N-Oxid und/oder in das Säureadditionssalz einer physiologisch verträglichen Säure überführt.

8. Therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln.

9. Verbindungen der Formel I zur Verwendung bei der Bekämpfung von Krankheiten.

Europäisches Patentamt

# EUROPÄISCHER RECHERCHENBERICHT

Nummer der Anmeldung

EP 88107665.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,A | DE - A1 - 3 524 744 (BASF AG) * Ansprüche 1,2,10 * -- | 1,2,7-9 | C 07 D 495/04<br>A 61 K 31/55 |
| D,A | DE - A1 - 3 037 971 (BASF AG) * Ansprüche 1,2,3,11,12 * -- | 1,2,7-9 | |
| D,A | DE - A1 - 2 918 778 (BASF AG) * Ansprüche 1,14,15 * ---- | 1,2,7-9 | |

RECHERCHIERTE SACHGEBIETE (Int. Cl.4)

C 07 D 495/00

Der vorliegende Recherchenbericht wurde für alle Patentansprüche erstellt.

| Recherchenort | Abschlußdatum der Recherche | Prüfer |
|---|---|---|
| WIEN | 03-08-1988 | BRUS |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82